# EUROPEAN PATENT APPLICATION

(11) **EP 4 379 077 A1**
(43) Date of publication of application: **05.06.2024**
(21) Application number: 22848747.6
(22) Date of filing: 26.07.2022
(51) Int. Cl.: C22B 30/06, B82Y 30/00, B82Y 40/00

(54) **2D ANISOTROPIC BISMUTH MATERIALS AND METHOD FOR OBTAINING SAME USING COLLOIDAL SYNTHESIS**

(30) Priority: 26.07.2021 ES 202130722
(71) Applicant: Universitat de València, 46010 Valencia (ES)
(72) Inventor: DOLLE, Christian, 46010 Valencia (ES); OESTREICHER, Victor, 46010 Valencia (ES); ABELLAN SAEZ, Gonzalo, 46010 Valencia (ES)
(74) Representative: Elzaburu S.L.P.
(86) International application number: PCT/ES2022/070492
(87) International publication number: WO 2023/007051

(57) **Abstract**

The present invention relates to 2D bismuth material, also called bismuthene, with a sandwich-like sheet structure with at least two outer layers formed by organic molecules containing sulphur atoms that form Bi-S bonds and at least one inner layer formed by a crystalline network of Bi(0) atoms. These materials are useful in electronic, optoelectronic, catalytic applications or in energy storage and transformation. Furthermore, it relates to a process for producing this material from a bismuth salt that reacts with an amine and subsequently with a thiol by effect of the application of radiation and a subsequent reduction. This process has a colloidal approach to producing Bi(0) crystals based on a photocatalytic reduction of a soluble Bi(III) organometallic complex leading to the generation of said crystals.

## Description

### TECHNICAL FIELD OF THE ART

The present invention relates to a two-dimensional (2D) bismuth material, also called bismuthene, with a sandwich-like sheet structure with at least two outer layers formed by organic molecules with sulphur atoms and at least one inner layer formed by a crystalline network of Bi(0) atoms. It also relates to a process of obtaining this material based on a colloidal approach wherein a photocatalytic reduction of a soluble Bi(III) organometallic complex is performed leading to the generation of the Bi(0) crystal lattice. Therefore, the present invention belongs to the field of nanomaterials based on pnictogens and their production processes.

### BACKGROUND OF THE INVENTION

Post-graphene sheet materials are of great scientific interest due to their unprecedented physical and chemical properties as they approach the two-dimensional (2D) limit. These systems could be promising not only for (opto)electronic applications, but also for catalysis, energy storage or biological applications. Among all ultra-thin sheet materials, 2D pnictogens occupy a prominent place since their properties can complement or even surpass graphene in some cases. Both P and As, Sb and Bi have sheet allotropes that can be exfoliated. However, the generation of anisotropic thin layers is increasingly complicated as we go down in the periodic table. This is due to the increasing inter-layer forces present on increasing atomic weight (P<As<Sb<<Bi). This results in a drastic reduction in lateral dimensions and poorly defined morphologies when exfoliations are performed in the liquid phase, as well as in very low or zero degrees of exfoliation when micromechanical exfoliation is used. Of all 2D pnictogens, bismuth is the most difficult to exfoliate. Hence, obtaining 2D pnictogens, especially from bismuth, represents a very significant challenge.

The scalable synthesis of high-quality 2D pnictogens in general, and bismuth in particular, represents a major challenge for the scientific community (Applied Physics Reviews 6, 021308 (2019)). Nowadays, the most common strategies are liquid phase exfoliation and epitaxial growth. Liquid phase exfoliation is the typical approach, although, in the case of bismuth, this technique causes a huge reduction in lateral dimensions mainly due to the strong interactions between the layers, as well as a pronounced oxidation, affecting both the final properties and the use in actual devices. The epitaxial growth technique is extremely expensive and does not allow independent crystals to be obtained, which prevents a mass production of these materials. In both cases, the processes mainly lead to the generation of small nanoparticles of different size and high polydispersity.

The document J. Phys. Chem. B, Vol. 110, No. 51, 2006, describes the synthesis of nanocubes, triangular nanoplates, nanobelts and Bi spheres using NaBiO₄, polyvinylpyrrolidone and Fe(III), although these materials cannot be considered two-dimensional. The document Nanoscale, 2018, 10, 21106-21115*,* describes the acid exfoliation of coarse Bi using ammonium peroxydisulfate ((NH₄)₂S₂O₈), H₂SO₄ and H₂O₂, which results in particles without form below 500 nm, without control over morphology and highly defective.

Angew. Chem. Int. Ed. 2011, 50, 1363 -1366, describes the synthesis of Bi nanocrystals by the reduction of bismuth dodecanethiolate, which was generated by the reaction of dodecanethiol and bismuth neodecanoate in octadecene, and the subsequent reduction with tri-n-octylphosphine (TOP). J. Phys. Chem. C 2014, 118, 2, 1155-1160, describes a process for producing bismuth nanospheres by thermolysis of bismuth acetate in oleylamine. But 2D materials are not obtained in any of these cases.

There is, therefore, a need to have efficient processes for the synthesis of 2D pnictogen crystals, and particularly Bi, which allow their growth and morphology to be controlled and which are easily scalable.

### DESCRIPTION OF THE INVENTION

The present invention relates to 2D bismuthene material, with a sandwich-like sheet structure with at least two outer layers formed by organic molecules containing sulphur atoms and at least one inner layer formed by a crystalline network of Bi(0) atoms. These anisotropic crystals of Bi(0) have more than 200 nm of lateral dimension and a high size/thickness ratio. The formation of covalent bonds between the sulphur atoms of the outer layers and the most adjacent Bi(0) atoms induces a stability that results in resistance to degradation in the open atmosphere. The material characteristics given by this particular structure make it especially useful in catalytic, magnetic and opto-electronic applications.

Furthermore, the present invention relates to a process for producing the aforementioned 2D bismuthene material. Said process has a colloidal approach based on a photocatalytic reduction of a soluble organometallic complex Bi(III) leading to a generation of said crystals. This simple synthetic approach allows the manufacture of 2D bismuthene, paving the way to monolayer crystals, and can be easily scaled up to use these 2D crystals in the implementation of interesting medical applications, topological properties, and catalysis, to name a few.

More specifically, the present invention discloses the colloidal synthesis for obtaining anisotropic 2D crystals of Bi(0), also called bismuthene, based on the photochemical reduction of an organometallic complex of Bi(III)-dodecanethiol. The role of reaction variables (dodecanethiol (DDT) concentration, temperature, intensity, and colour of light), the effect on kinetics, and crystal size and morphology were evaluated and the results are shown below. After optimising the conditions, micrometric crystals (>1.5 µm) of rhombohedral Bi(0), with a thickness less than 10 nm, preferentially exhibiting the [001] plane, can be easily obtained. The meticulous structural, spectroscopic and chemical characterisation makes it possible to assert that the crystals are single crystals, without defects on surfaces greater than 1000 nm², with the absence of oxidation and presenting a thin layer of thiol (easily removable) that protects it against oxidation.

While two-dimensional pnictogens, especially phosphorene, tend to oxidise strongly and, therefore, to decompose under ambient conditions, the material of the invention has enormous stability to oxidation that is demonstrated by comparing the Raman spectra of individual hexagons in silicon wafers with a layer of silicon oxide directly after synthesis and after several days (Example 3). This stability is the direct result of a surface coating based on the synthesis process of the present invention.

Formation of the 3*C*₁₂*H*₂₆*SH* + *Bi*(*C*₁₀*H*₁₉*O*₂)₃ → *Bi*(*C*₁₂*H*₂₆*S*)₃ + 3*C*₁₀*W*₂₀*O*₂ organometallic complex

### Photochemical reaction used to obtain Bi(0)

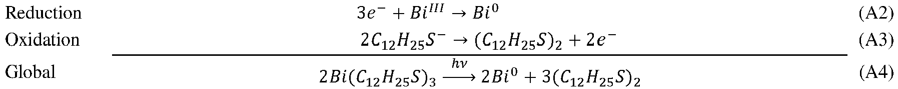

It is known that 2D pnictogens tend to undergo severe degradation in an open atmosphere. For example, low-layer phosphorene and arsenene oxidise completely within hours. However, this limitation is overcome with the synthesis process of the invention, since the low-layer bismuthene is protected by a surface coating. The oxidation stability of the resulting material is demonstrated by the structural characterisation of the fresh individual bismuthene hexagons compared to the individual hexagons characterised a few days later, without any signs of ageing.

Therefore, in a first aspect the present invention relates to a material comprising two-dimensional crystals of Bi(0) characterised in that it is formed by:
- at least two outer layers A comprising organic molecules R₂-SH, with R₂ being optionally substituted linear or branched C₁-C₁₈ alkyl or optionally substituted C₅-C₁₀ aryl,
- and at least one inner layer B between layers A comprising metal Bi(0) atoms forming a crystal structure,
wherein the S atoms of the organic molecules R₂-SH of layers A are covalently bonded to the adjacent Bi(0) atoms of layer B and wherein said layers A and B are arranged forming a sandwich-like sheet structure with anisotropic order on the stacking axis.

The crystals that form this material are two-dimensional hybrid macrocrystals (diameter in the order of microns, thickness on the nanometer scale). The outer layers A are functionalised with the sulphur atoms of R₂-SH and the inner layer B is rhombohedral bismuth, understanding functionalisation as the incorporation of functional groups that can form covalent bonds and other types of bonds with the molecules present in the structure and which favours the incorporation of other molecules or other functional groups to said structure, giving it new properties and/or functionalities.

In a preferred embodiment, layers A have a thickness of between 0.5 to 3 nm. In a more preferred embodiment, layers A have a thickness of 1.5 nm.

In a preferred embodiment, layer B has a thickness of between 5 to 10 nm. In a more preferred embodiment, layer B has a thickness of 7 nm.

In another preferred embodiment, the Bi(0) of layer B has a rhombohedral crystal structure (PDF #44-1246).

In another preferred embodiment, the material of the invention forms crystals of hexagonal morphology. These crystals preferably have a diameter greater than 1,000 nm, and may be more preferably up to 10-25 microns, and/or a thickness of less than 20 nm, and may be more preferably up to 3 nm, and/or an aspect ratio greater than 500.

In another preferred embodiment, the outer layers A have lower density than the inner layer B.

In a second aspect, the present invention relates to a process for producing the above-described Bi(0) nanocrystal material comprising the following steps:
a) preparing a solution of a bismuth salt with the following formula:

   [R₁-COO-]Bi³⁺ (I)

   wherein R₁ is a linear or branched C₁-C₁₈ alkyl,
   in an organic solvent with the following formula:

      CH₃-(CH₂)ₘ-CH=CH₂ (II)
   where m is an integer value selected from 1 to 20,
   and add an amine with the following formula:

      NH₂-(CH₂)ₙ₁-CH=CH-(CH₂)ₙ₂-CH₃ (III)
   where n1 and n2 are integer values independently selected from between 1 and 10,
b) increasing the temperature of the reaction mixture obtained in (a) to a temperature of between 150-250 ºC, subjecting to vacuum and applying radiation with a wavelength of between 430 and 530 nm,
c) breaking the vacuum of the reaction medium of (b) by the introduction of an inert gas,
d) adding to the reaction mixture a reducing agent with the following formula:

   R₂-SH (IV)

   wherein R₂ is selected from optionally substituted linear or branched C₁-C₁₈ alkyl or optionally substituted C₅-C₁₀ aryl,
e) stopping the reaction and separating the product obtained.

The term "alkyl" refers, in the present invention, to hydrocarbon chain radicals, linear or branched, having between 1 and 18 carbon atoms, being bound to the rest of the molecule by a single bond, for example, but not limited to, methyl, ethyl, *n*-propyl, *i*-propyl, butyl, *tert*-butyl, *sec*-butyl, *n*-pentyl, dodecyl, etc. These alkyl groups can be substituted by one or more substituents such as halogens, hydroxyl, alkoxy, carboxyl, carbonyl, cyano, acyl, alkoxycarbonyl, amino, nitro, mercapto and alkylthio.

The term "aryl" refers, in the present invention, to single or multiple aromatic rings, having from 5 to 10 bonds wherein a proton has been removed from the ring. Preferably, the aryl group has 5 to 7 carbon atoms. The aryl groups are, for example, but not limited to phenyl, naphthyl, diphenyl, indenyl, phenanthryl or anthracyl. The aryl radicals may be optionally substituted by one or more substituents such as halogens, hydroxyl, alkoxy, carboxyl, carbonyl, cyano, acyl, alkoxycarbonyl, amino, nitro, mercapto, and alkylthio.

In a preferred embodiment, in the bismuth salt of formula (I), R₁ en is a linear or branched C₈ alkyl.

In a more preferred embodiment, the bismuth salt of formula (I) is as follows:

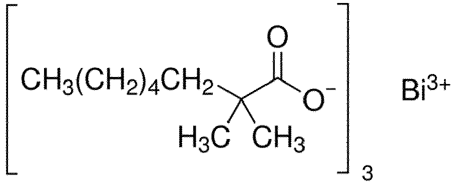

In another preferred embodiment, in the organic solvent of formula (II) m is an integer value selected from between 10 and 18.

In a more preferred embodiment, the organic solvent of formula (II) is as follows:

CH₃(CH₂)₁₅CH=CH₂

In another preferred embodiment, in the amine of formula (III) n1 and n2 are an integer value independently selected from between 5 and 8.

In a more preferred embodiment, the amine of formula (III) is as follows:

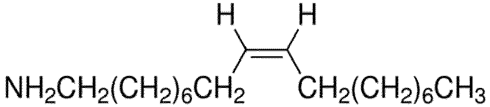

In another preferred embodiment, the temperature in step (b) is 200°C.

In another preferred embodiment, the radiation of step (b) is applied for a time of between 10 and 30 minutes, preferably 15 minutes.

In another preferred embodiment, the inert gas of step (c) is a non-oxidising gas. More preferably, it is selected from argon, nitrogen or carbon dioxide.

In another preferred embodiment, in the reducing agent of formula (IV), R₂ is selected from a linear C₈-C₁₂ alkyl or a phenyl. More preferably, the reducing agent of formula (IV) is selected from dodecanethiol or thiophenol.

In a preferred embodiment, the reaction is stopped in step (e) by a sudden decrease in temperature that can be performed with an ice bath.

In another preferred embodiment, the product is separated in step (e) by centrifugation.

Another aspect of the invention relates to the use of the two-dimensional Bi(0) nanocrystal material described above for energy storage and generation, for example in Li, Na or K batteries; for electrocatalysis or organic catalysis; for obtaining (opto)electronic materials; for photonic applications; in contrast agents for diagnostic tests.

### BRIEF DESCRIPTION OF THE FIGURES

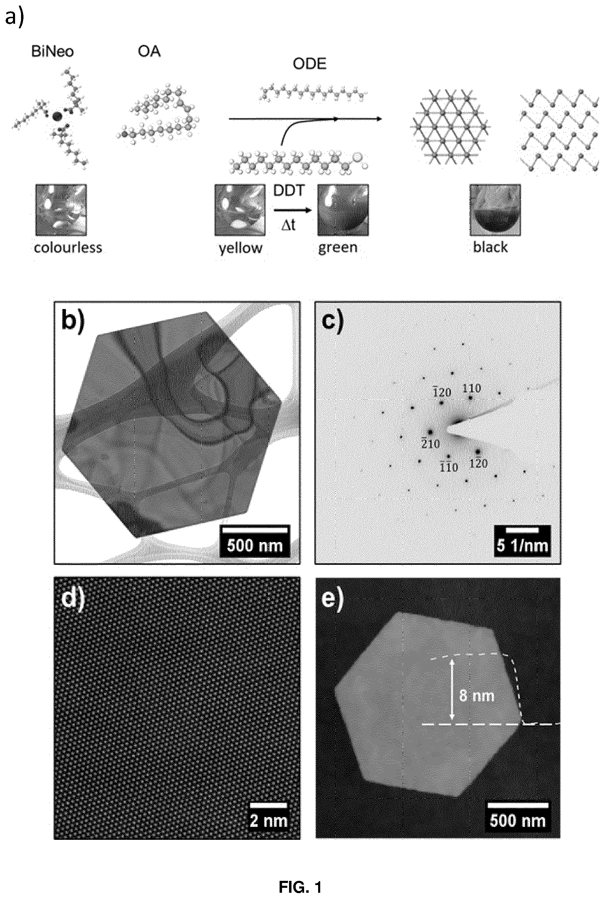

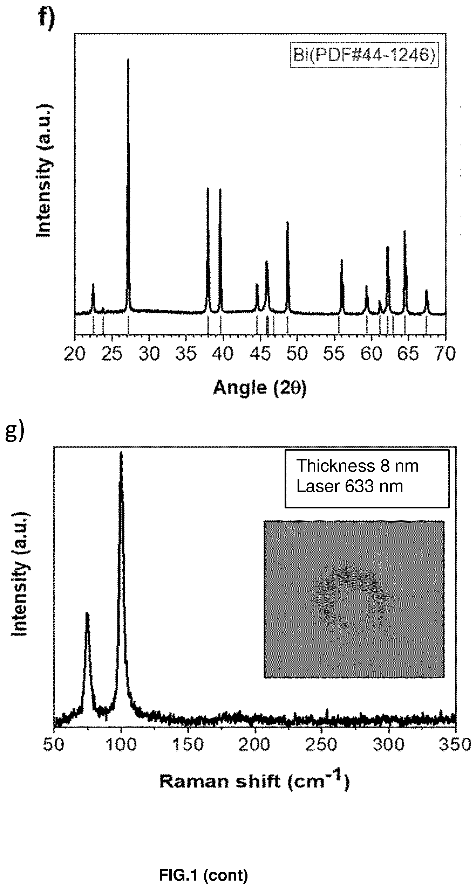

Figure 1.- (a) Shows the synthetic procedure for obtaining a few layers of two-dimensional bismuthene (2D). A solution of BiNeo and OA in ODE is degassed at 200°C, then DDT is added to the reaction mixture. The formation of the organometallic complex Bi-DDT is demonstrated by the yellow colour of the solution. Subsequently, the Bi(III) is reduced to Bi(0) while the thiol is oxidised to disulfide. *(b*) BF-TEM of a single crystal of Bi(0) with a diameter greater than 1.5 µm, *(c)* electron diffraction (SAEP) that allows observing the rhombohedral ordering along the axis [001]. (*d*) High-resolution TEM image. *(e)* AFM performed in *tapping* mode indicating the scale on the z-axis. *(f)* PXRD pattern that enables corroborating in general terms the obtaining of Bi(0) with rhombohedral structure (PDF#44-1246). *(g)* Single crystal Raman spectrum (inset shows optical micrograph), recorded with red laser (633 nm), exclusively showing peaks E_{g} and A_{1g}.

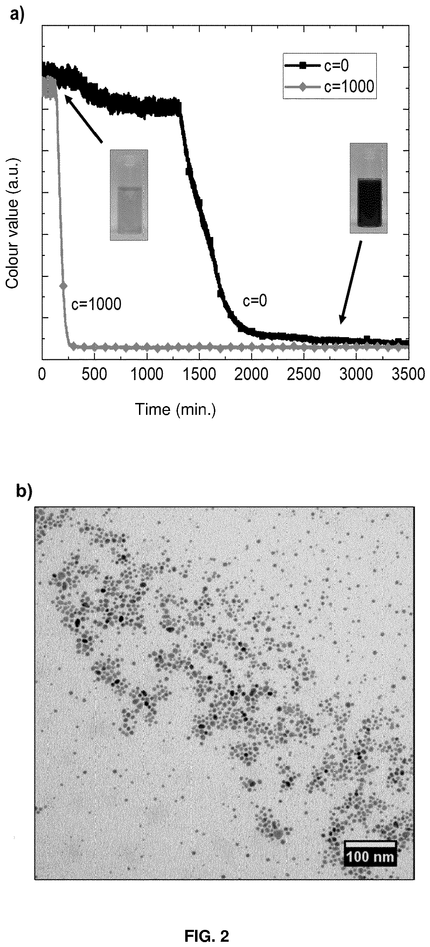

**Figure 2****.-** *a*) Dependence of the reaction rate on DDT concentration. The black curve, 3 equivalents of DDT with respect to Bi(III), while the grey curve has an excess of DDT (1000 mM). Photographs of the solutions at the beginning (yellow. Bi-DDT) and end (black. Bi(0) NPs) of the reaction. *b*) TEM image that enables observing the presence of Bi(0) NPs with spherical morphology.

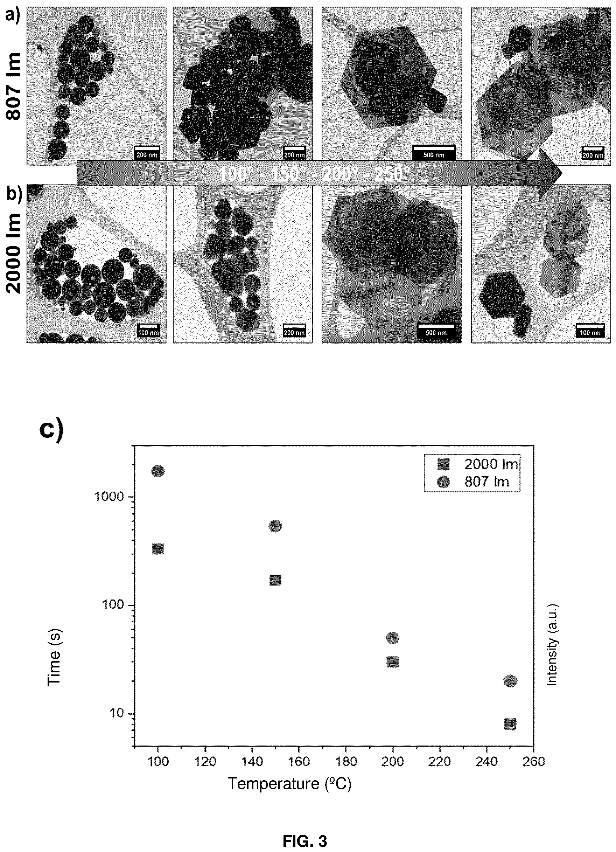

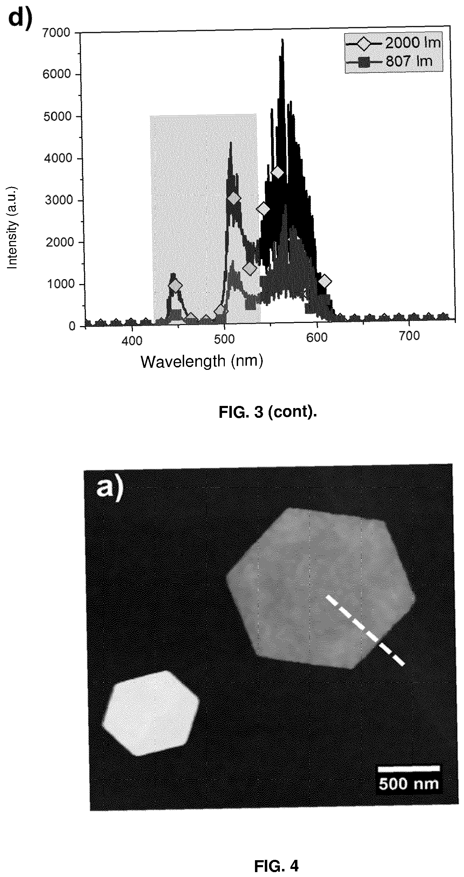

**Figure 3****.-** Dependence of light and temperature on producing Bi(0) by photochemical reduction. Representative BF-TEM images of the nanomaterials obtained for the different temperatures evaluated using white LED light of different intensity: 807 Im (*a*) and 2000 Im (*b*). Dependence of reaction rate on temperature and light intensity or brightness (*c*). Characteristic emission spectra of the LED lamps used: 807 Im (grey) and 2000 Im (black) (*d*). The grey box indicates the region of the electromagnetic spectrum responsible for the photocatalytic reaction, 450-550 nm.

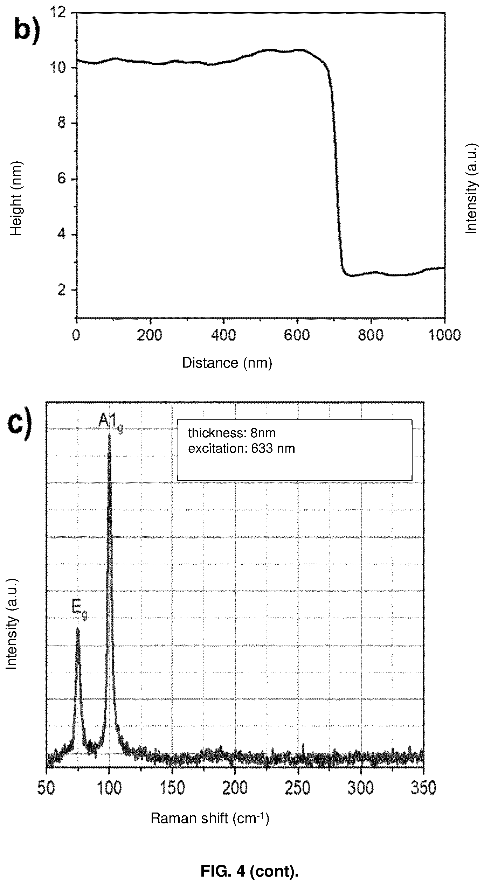

**Figure 4****.-** AFM image of a hexagonal particle *(a)* wherefrom the thickness profile (*b*) is extracted. Raman spectrum typically obtained with the signals E_{g} and A_{1g} (*c*).

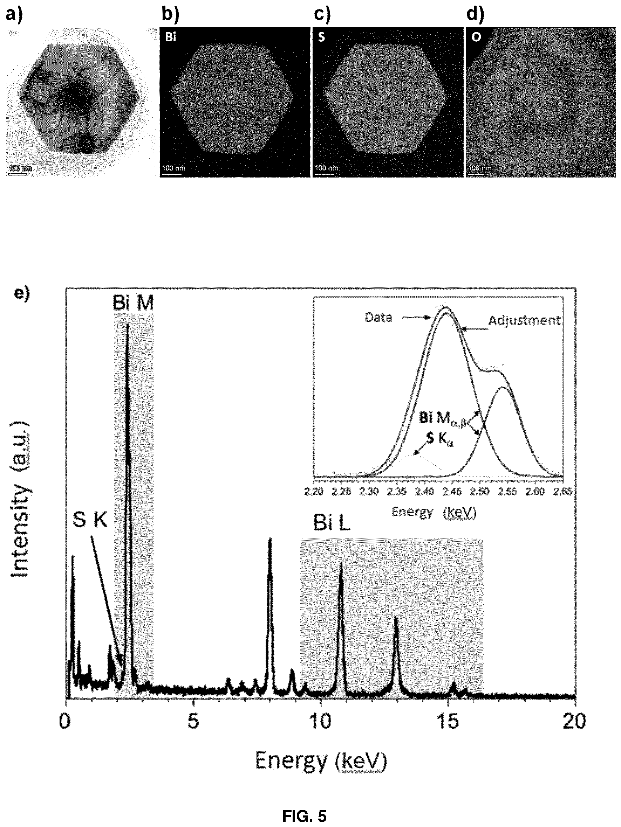

**Figure 5****.-** BF-STEM image (*a*) and integrated EDS signals for Bi (*b*), S (*c*) and O (*d*). EDS spectrum signalling lines Bi-M, Bi-L and S-K (*e*). Adjustment of the EDS signal in the region of energy comprised for the Bi-M and S-K lines considering the sulphur signal.

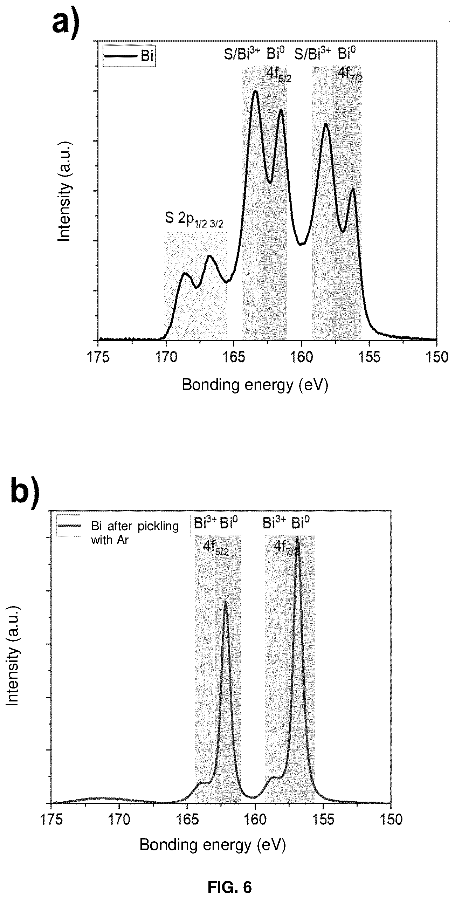

**Figure 6****.-** XPS spectrum of the material obtained by the synthesis process where a clear characteristic signal of S 2p is observed that is superimposed with the Bi 4f signals. (*a*). After removal of this S-containing surface layer by Ar⁺ pickling, no S signals are observed and instead Bi(0) signals can be observed exclusively *(b)*.

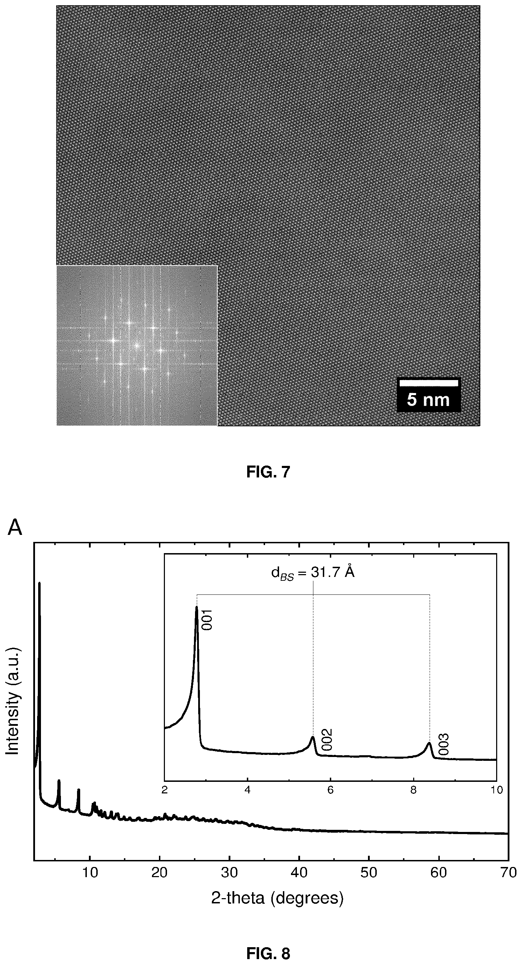

**Figure 7****.-** AC-HAADF-HRSTEM image of Bi(0) where a defect-free surface can be observed on a scale greater than 1000 nm². An FFT of the image is shown in the box.

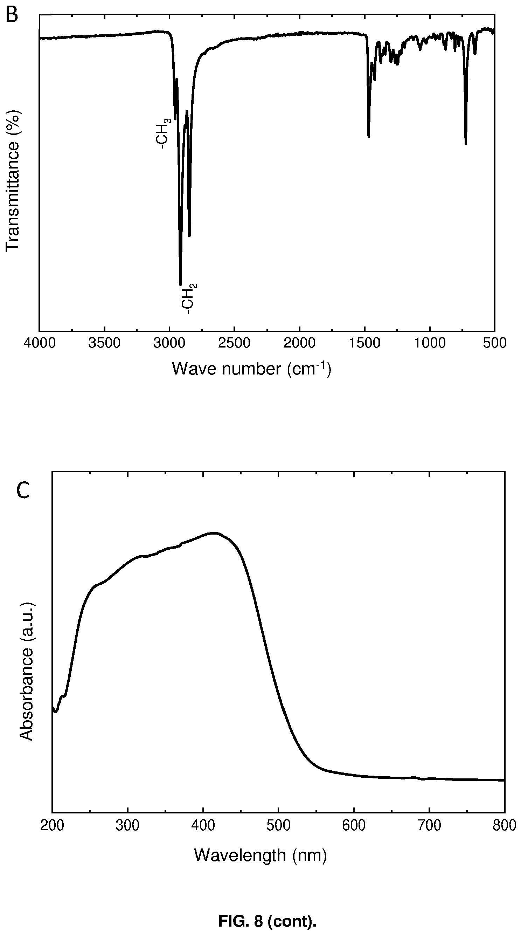

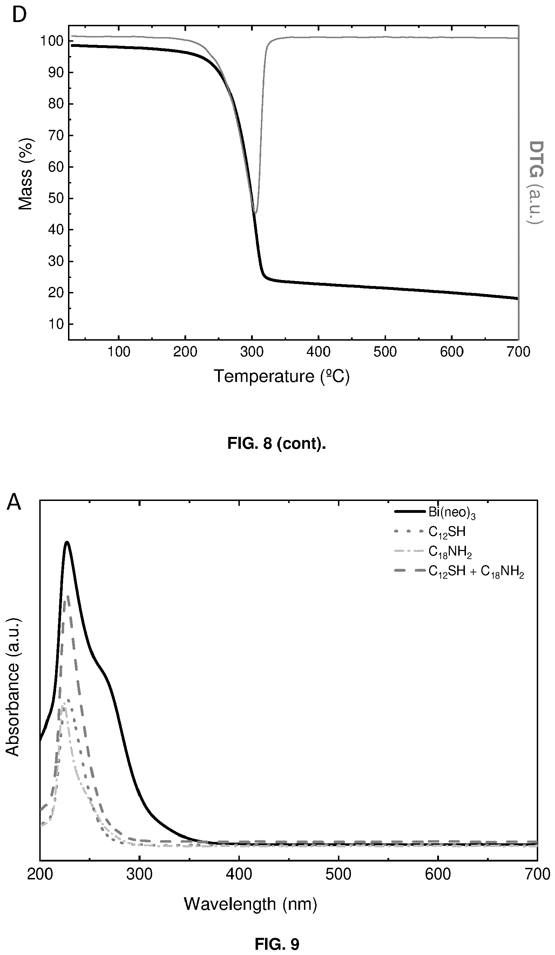

**Figure 8****.-** Characterisation of the organometallic precursor of Bi(III): PXRD pattern . *Box*: increase in the 2-10º range. The first three peaks can be indexed with a *00I* group with an interlamellar distance of 31.7 Å (A). ATR-FTIR spectroscopy. The absence of signals at 2500 cm⁻¹ (S-H vibrations) ensures the absence of free thiol, suggesting Bi-S bonds (B). The diffuse UV-Vis reflectance spectrum has an absorption maximum at 417 nm (C). Thermogravimetric analysis carried out in an inert atmosphere (He, 20 mL/min) at 10 ºC/min, enables observing a single decomposition step at 305 ºC (D).

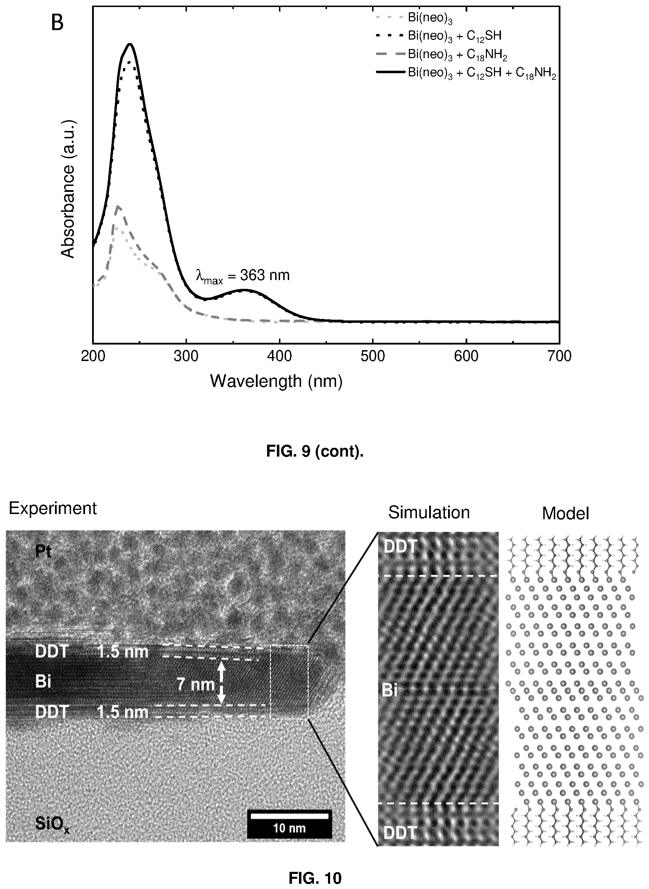

**Figure 9****.-** UV-Vis absorption spectra for solutions containing mixtures of Bi-Neo, DDT and amine: pure reagents (A) and mixture thereof (B).
**Figure 10****.** Cross-section inspection of hexagonal particles obtained by the method of the invention. A sandwich structure based on the surface layer of ca. 1.5 nm and a core section of approx. 7 nm. Experiment (left), simulation (centre) and model (right).

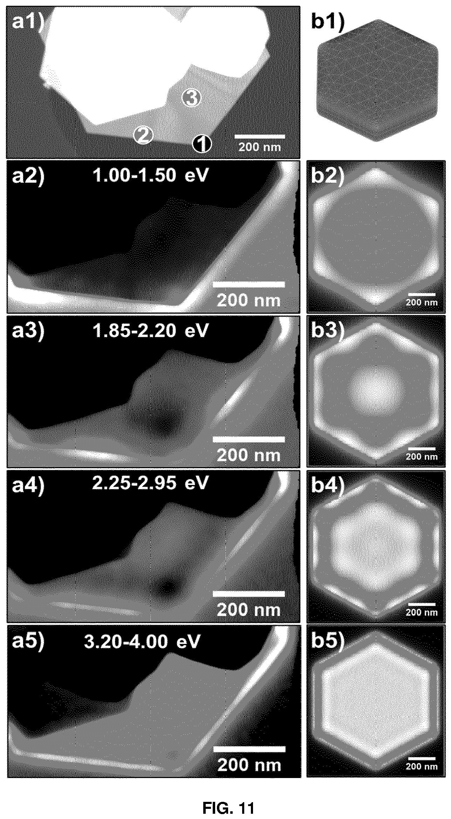

**Figure 11****.** Localised surface plasmons of colloidally synthesised few-layer bismuthene. a) STEM-EELS experimental data: a1) HAADF-STEM data with the positions indicated for the extraction of EEL spectra. a2)-a5) integrated EELS intensities showing 4 distinct plasmonic modes in the indicated energy ranges. b) Simulation of the EELS intensities of individual hexagons.

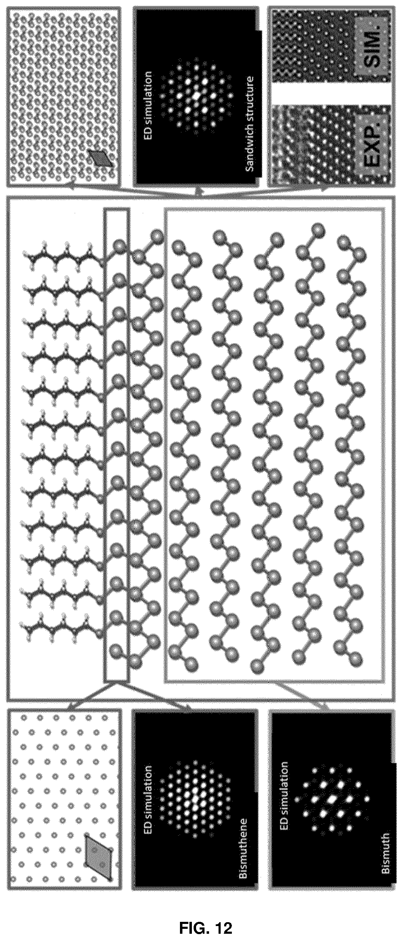

**Figure 12****.** Detailed schematic representation of the bismuth/bismuthene sandwich hybrid structure. Electron diffraction patterns for each of the indicated regions. (lower right panel) Inspection of the cross-section by means of HRTEM compared to its respective simulation.

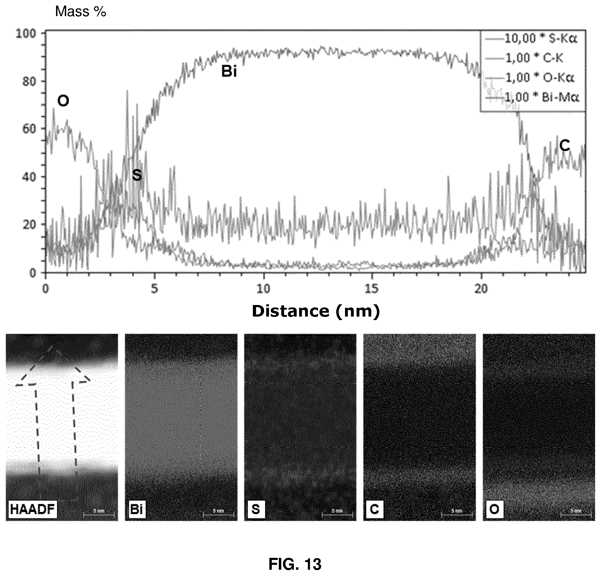

**Figure 13****.** Inspection of the cross-section of hexagonal particles obtained by the method of the invention by STEM coupled with EDS/EDX probe. Upper panel: The lines represent the mass normalised percentage for the Bi-M, SK, C-K and O-K signals. Note: the S-K signal was increased by a factor of 10x to expedite viewing. The lines corresponding to the signals of each element are extracted from the HAADF image (lower panel), then allowing a visual inspection of the atomic distribution.

### EXAMPLES OF THE INVENTION

### Example 1: Synthesis of Bi2D

In this process, a soluble organic precursor of Bi(III) was reduced under the influence of light and temperature, using dodecanethiol as a reducing agent. The obtained material was separated from the reaction mixture by centrifugation and washed with chloroform.

As a first step, a stock solution of 100 mM bismuth neodecanate (BiNeo) in 1-octadecene (ODE) was prepared by dissolving 3.614 g BiNeo in 25 mL ODE. In the particular case of colloidal synthesis, 125 µmol (1.25 mL) of this solution were pipetted and placed in a 50 mL two-necked flask. Subsequently, 22.22 mL of ODE and 1.235 mL of oleylamine (3.75 mmol, 30 eq.) were added. The system was subjected to vacuum, while being heated in oil bath (200 °C) under constant magnetic stirring (400 RPM) and continuously illuminated using an LED lamp. After 13 minutes of degassing, it was changed to argon atmosphere, while the reaction mixture remained at 200 °C for a further 2 minutes. Through a septum, and using a syringe, 0.3 mL (1.25 mmol, 10 eq.) of dodecanethiol (DDT) was injected. The resulting mixture was allowed to react for 30-40 seconds. Upon addition of DDT, the reaction mixture immediately turned yellow, indicating formation of Bi-DDT. The organometallic complex of Bi(III) was photochemically reduced to Bi(0) while DDT was oxidised to disulfide. The reduction of Bi(III) to Bi(0), and consequently the end of the reaction, was confirmed by the appearance of a black colour (see colour evolution in the scheme of Fig. 1a), showing a marked nematic texture (a first evidence of the high anisotropy of crystals, i.e.: two-dimensional (2D) crystals). The reaction was stopped by immersing the flask in ice bath for 5 minutes. The solution was then centrifuged (10k RPM, 10 minutes) in an argon dry box to avoid oxidation of the Bi(0) crystals.

After separation of the 2D crystals of Bi(0), the solid was redispersed in chloroform and washed by centrifugation 3 times (10 k RPM, 10 minutes). In this way, we sought to exchange the remaining ODE with chloroform, thus allowing the solid to dry more easily.

The final product was allowed to dry in the dry box, or stored as a suspension in chloroform, to be used in subsequent characterisations. In the case of microscopies, the suspensions were sonicated before use (TEM, SEM, AFM, Raman), while in other assays the final dry solid was used (PXRD, TGA).

**Table 1. Chemical reagents, molecular formulas and codes. Note: Bismuth dodecanethiolate is the organometallic complex of Bi(III) referred to throughout the text.**

| **Name** | **Formula** | **Code** |
|---|---|---|
| Bismuth neodecanoate | *Br*(*C*₁₀*H*₁₉*O*₂)₃ | *Bi*(*neo*)₃ |
| Bismuth dodecanethiolate | *Br*(*C*₁₂*H*₂₅*S*)₃ | *Br*(*C*₁₂*S*)₃ |
| Dodecanethiol | *C*₁₂*H*₂₅*SH* | *C*₁₂*SH* |
| Dodecyldisulfide | (*C*₁₂*H*₂₅*S*)₂ | (*C*₁₂*S*)₂ |
| Oleylamine | *C*₁₈*H*₃₅*NH*₂ | *C*₁₈*NH*₂ |
| Octadecene | *C*₁₈*H*₃₆ | *C*₁₈ |

### Example 2: Characterisation of 2D Bismuth Crystals

Figure 1 shows the microscopic and structural characterisation of the 2D hexagonal crystals of Bi(0) obtained. In Figure 1b) a low magnification image of BF-TEM enables observing crystals of around 1.5 µm in diameter exhibiting hexagonal morphology. In Figure 1c), the electron diffraction pattern (SAEP) provides clear evidence of a rhombohedral crystal structure, where the crystallite size is oriented on axis [001], along the stacking axis of the lamellar crystal. HRTEM (d) and AFM (e) confirm the hexagonal morphology of the obtained lamellar crystals. The PXRD pattern in (f) can be perfectly indexed with the crystal structure reported for rhombohedral Bi (PDF#44-1246), providing a final test of the overall rhombohedral structure of the crystals obtained.

The organometallic complex Bi-DDT was identified as the active species (precursor) in the reduction reaction from Bi(III) to Bi(0), which can be monitored even at room temperature (see Example 3 below). In Figure 2a) the dependence of the reaction rate on the concentration of DDT is observed. When using the stoichiometric amount of DDT (3 equivalents with respect to Bi(III), black curve) the reaction is considerably slower than when using an excess of 1000 mM of DDT (grey curve). Beyond the concentration of DDT used, in all cases evaluated, spherical nanoparticles smaller than 20 nm in size are obtained at room temperature (see Figure 2b). It is important to mention that the reaction indispensably needs visible light for the reduction of Bi-DDT to Bi(0) to take place. If the Bi-DDT precursor, or the reaction mixture, is stored in the dark the reduction does not occur.

Taking into account the behaviour at room temperature, the dependence of the reaction rate and the resulting morphology on both the light intensity and the reaction temperature was also evaluated. The results are shown in Figure 3. Overall, a clear trend between reaction rate and temperature can be observed. At higher temperatures, the time needed to reduce Bi(III) to Bi(0) decreases considerably, as can be seen in Figure 3c) (note that the time axis is on a logarithmic scale). In addition to the effect of temperature on reaction rate, a marked increase in reaction rate can also be observed when brighter/intense LED lamps are used (black squares, 2000 lm vs. grey circles, 807 lm). Figure 3d) shows the emission spectrum of the lamps where, beyond the change in intensity, it can be observed that both emissions are similar, thus ruling out the effect of changes in the emission spectrum. In turn, experiments were performed using red and blue light, which allow us to conclude that only the blue emission is responsible for increasing the reaction rate (grey box, Figure 3d). Interestingly, red light can even slightly slow down the reaction rate.

As can be seen in Figure 3a), temperature plays a key role in the morphology of Bi(0) crystals. While spheroidal particles prevail at low temperatures, the increase in spheroidal particles favours the faceting of the particles that eventually develop into highly anisotropic 2D materials at temperatures of 200ºC or higher. Interestingly, in the case where the maximum temperature and the most intense light are used, the size of the crystals decreases again, observing a growth along the most pronounced stacking axis (thickness).

After this analysis, the parameters 200 ºC and 2000 lm were chosen as the optimised synthetic conditions. Figure 4 shows an AFM image a) indicating the sector where the thickness measurement b) was taken. The Raman spectrum of an 8 nm thick crystal is shown in Figure 4c), where the typical double degenerate in-plane and out-of-plane modes can be observed *with* symmetries E_{g} and A_{1g} with Raman displacements of 74.8 and 99.8 cm⁻¹, respectively. Spectra were measured using a HeNe laser with an emission wavelength of 632.8 nm. The characterisation performed by Raman microscopy enables ruling out the presence of Bi₂O₃ (signals around 320 cm⁻¹), guaranteeing the absence of oxidation in the Bi(0) crystals.

STEM-EDS mappings reveal the elemental composition of the hexagonal crystals of Bi(0) with a small amount of sulphur (S) on the surface. Figure 5b-d) shows the mapping of the elements Bi, S and O. While Bi and S are equally distributed, the absence of O in the crystal guarantees the purity of the crystals obtained (native surface without oxidation). Figure 5e) shows the integrated EDS spectrum indicating the S-K line. Since the sulphur signal overlaps considerably with the Bi-M lines, an adjustment of the EDS signal was made in the energy range 2.20-2.65 keV. Results are shown in Figure 5f-g. In this way it is clear that a quantitative adjustment of the EDS signal can only be made if the intensity of the S-K line is considered.

XPS measurements confirm the presence of sulphur on the surface of the Bi crystals. Analogous to the case of EDS measurements, the energies of Bi 4f and S 2p overlap, preventing an unequivocal characterisation of the surface chemistry of the crystals (Figure 6a). However, after performing a pickling using Ar+ ions to remove the first surface layers, the characteristic Bi(0) signal can be unequivocally observed (Figure 6b). This first surface layer (s) is rich in sulphur and acts as a barrier against oxidation, preserving the identity of the material.

Figure 7 shows an image of HAADF-HRSTEM with aberration correction, where in turn, a fast Fourier transform, FFT (Fast Fourier Transformation) is observed. As noted, the material is a single crystal without considerable defects on a large scale (>1000 nm²).

### Example 3: Surface Chemistry

In particular, more detailed research was performed using spectroscopic techniques, namely XPS and STEM-EDS in combination with Raman spectroscopy. In Figure 5, the STEM-EDS mapping shows the integrated Bi-M, S-K, and O-K signals next to a BF-STEM image. The higher magnification TEM data contained in ED show a thin amorphous edge around the Bi crystallites that are indicative of the thickness of the coating layer.

From the EDS it follows that Bi and S are equally distributed, while the oxygen and carbon signal can be clearly correlated with the underlying support film of the TEM. As shown in the inset of Figure 5e, only the additional contribution of sulphur allows a correct adjustment of the obtained integrated signal. The surface sensitivity of X-ray photospectroscopy helps to clarify the presence of a sulphur-rich surface layer, as seen in Figure 6. Interestingly, the strong contribution of S 2p between 165-170 eV points towards strongly oxidised sulphur species (BiS bond test), indicative of oxygen protection.

Under vacuum conditions in the XPS, a brief sputtering of Ar (30 s) was performed to remove this coating layer with the result shown in FIG. 6 a and b. After etching, an almost complete loss of the XPS intensities related to carbon (285 eV) and oxygen (532 eV) can be followed, as well as the disappearance of the S 2p contribution between 165-170 eV (Figure 6b). By focusing on the Bi 4f region, a shift of both 4f_{7/2} and 4f_{5/2} contributions is evident, giving rise to a pure Bi(0) surface. Therefore, Ar sputtering allows simple and effective removal of the protective layer and gives easy access to pure non-oxidised Bi(0).

The combination of surface sensitive XPS and spatially resolved EDS thus allows concluding the equally distributed surface coating with the protective sulphur species which, while separating Bi from environmental degradation, can be easily removed by ion beam sputtering.

Finally, a cross-sectional inspection of the hexagonal microparticles has been performed to unequivocally demonstrate the sulphur-based protective layer. Figure 10 shows the TEM image recorded on a hexagonal particle. As can be seen, the material shows an ordering of the atoms throughout the inspected area. Interestingly, two different sections can be observed, namely the surface exhibits a different atomic distribution compared to the inner part. While in the core of the microparticle the structure corresponds to the rhombohedral Bi (as demonstrated by PXRD in Figure 1), the surface consists of a highly ordered arrangement of the Bi-S atoms, responsible for the lack of surface oxidation (Figure 5d).

Further analysis of this sandwich structure was performed by electron diffraction (ED). Figure 12 shows a more detailed description of the bismuth/bismuth hybrid structure with the ED simulation for each layer. The perfect correlation between the obtained ED patterns and the simulation reinforce the hypothesis about obtaining the structure that protects the present invention. Finally, an inspection of the cross-section of this hexagonal material obtained by the method of the invention was carried out by means of stem coupled with an EDS/EDX probe (Figure 13), which allows spatially locating each type of element. As can be seen in the upper panel, while in the centre of the particles it is only composed of Bi, it is on the faces where the presence of S and C atoms, typical of the DDT molecule, is observed. It is important to note that no O atoms are observed either inside or on the surface of the material, thus being able to rule out the formation of bismuth oxides of BiₓO_{y} type. Therefore, these results confirm the obtaining of a sandwich structure composed of a rhombohedral Bi core and two protective outer layers of functionalised Bi covalently bound to the DDT molecule (Bi-S bonds).

### Example 4: Surface plasmon modes

After clarifying the functionalisation/protection of the surface by spectroscopic techniques, monochromatic and aberration-corrected STEM-EELS spectrum images were used to investigate the plasmonic behaviour of the material in the low energy loss regime. Electronic excitations in unoccupied orbitals are usually probed in the EELS. Here the low-energy region of the material was investigated. The observed low energy modes can give access to phononic modes (< 1 eV), localised surface plasmon resonances (1-10 eV), as well as volume plasmon modes (-20-50 eV). These optical modes indicative of a metal surface are especially interesting for photonics and catalysis.

The observation of localised surface plasmons is a proof of the high quality of the surface, usually the quality of the plasmonic emission in bismuth systems is deteriorated by the accumulation of defects and distortions in the crystalline order. As shown in Figure 1, the colloidally synthesised hexagonal bismuth microparticles show no mentionable crystal defects, either locally (HR-STEM) or globally (PXRD), highlighting the advantages of the colloidal approach of the process of the invention.

This exceptional quality of the material allows viewing different plasmonic modes in an energy range below 10 eV. Figure 11a shows a STEM image of an investigated hexagon, the resolved modes (a2-a5), as well as the calculated field distributions (b2-b5). The extraordinary surface quality in combination with the next-generation electron microscopy thus allows the direct measurement of these surface modes without the need for any prior treatment.

Specifically, four different plasmonic modes could be displayed at the indicated energies. Comparison with the simulated results reveals an excellent match of the localisation near the surfaces. To extract the localised EELS spectra, a logarithmic Fourier deconvolution and a subsequent subtraction of the vacuum-acquired deconvoluted ZLP were performed.

### Description of the methodology in the measurements:

### Electron microscopy:

Transmission (scanning) electron microscopy and diffraction have been carried out on Bi(0) crystals placed on TEM sample holders covered with a lacey carbon film of a CHCl₃ solution. A JEOL-ARM200F was used for the AC-HRSTEM, operated at 200kV. TEM and ED measurements were carried out using a Tecnai F20 (200kV) or a JEOL JEM1010 (100kV). EDXS mappings were recorded on a Fei Titan Themis300 (300kV). Scanning Electron Microscopy data were acquired on a Hitachi S4800 FEG, operated at 10kV. The samples for the AFM, SEM and Raman measurements were prepared by pouring a Bi(0) solution onto silicon wafers with a standard silicon oxide layer and allowing the CHCl₃ to evaporate.

### Atomic force microscopy:

Atomic force microscopy was carried out using a Veeco Nanoscope IVa, operated in Si cantilever strike mode.

### Raman Spectroscopy:

Raman spectroscopic measurements were performed with a Horiba LabRam HR Evolution, using a HeNe laser (632.8 nm) with a final power of 7.6 µW and a 100x objective (NA 0.9).

### Powder X-ray diffraction:

Powder X-ray diffraction (PXRD) patterns were obtained using a PANalytical Empyrean X-ray platform with a capillary platform (diameter: 0.7 mm) and copper radiation (Cu Kα = 1.541 78 Å). Measurements were made in triplicate in the 2-theta 2-70° range using a step size of 0.02°/step with an integration time of 1 s.

### X-ray photoelectron spectroscopy (XPS):

XPS measurements were recorded on a Thermo Scientific^{™} K-Alpha X-ray Photoelectron Spectrometer. Al Kα X-ray radiation was used as the X-ray source. For all elements, more than 100 spectra were recorded using a 0.1 eV step with a focused point greater than 400 µm. XPS data were analysed with Thermo Avantage v5.9912 software.

### FIB cross section:

The FIB was carried out with a FEI Helios G4 dual-beam FIB-SEM. A representative crystallite was chosen for the cross-section and a protective layer of Pt was deposited over the crystallite. Two trenches were then cut into the silicon substrate with 30 kV Ga ions. The sheet was attached to the needle of a micromanipulator and lifted from the silicon wafer and welded to an Omniprobe copper grid using Pt. Then, the sheet was thinned until reaching electronic transparency (thickness of about 80 nm) with Ga ions. The foil sample was then investigated on a Philips CM200 FEG TEM, operated at 200 kV.

## Claims

1. Material comprising two-dimensional crystals of Bi(0) **characterised in that** it is formed by:
- at least two outer layers A comprising organic molecules R₂-SH, with R₂ being optionally substituted linear or branched C₁-C₁₈ alkyl or optionally substituted C₅-C₁₀ aryl,
- and at least one inner layer B between layers A comprising metal Bi(0) atoms forming a crystal structure,
wherein the S atoms of the organic molecules R₂-SH of layers A are covalently bonded to the adjacent Bi(0) atoms of layer B and wherein said layers A and B are arranged forming a sandwich-like sheet structure with anisotropic order on the stacking axis.

2. Material according to claim 1, wherein layers A have a thickness of between 0.5 to 3 nm.

3. Material according to claim 2, wherein layers A have a thickness of 1.5 nm.

4. Material according to any one of claims 1 to 3, wherein layer B has a thickness of between 5 to 10 nm.

5. A material according to claim 4, wherein layer B has a thickness of 7 nm.

6. Material according to any one of claims 1 to 5, wherein the Bi(0) of layer B has a rhombohedral crystal structure (PDF #44-1246).

7. Material according to any one of claims 1 to 6, wherein said material forms crystals of hexagonal morphology.

8. Material according to claim 7, wherein the crystals have a diameter greater than 1,000 nm.

9. Material according to any of claims 1 to 8, wherein the crystals have a thickness of less than 20 nm.

10. Material according to any of claims 1 to 9, wherein the crystals have an aspect ratio greater than 500.

11. Material according to any one of claims 1 to 10, wherein the outer layers A are functionalised with sulphur atoms that form Bi-S bonds and the inner layer B is rhombohedral bismuth.

12. Process for producing the material according to any of claims 1 to 11, which comprises the following stages:
a) preparing a solution of a bismuth salt with the following formula:
[R₁-COO⁻] Bi³⁺ (I)
wherein R₁ is a linear or branched C₁-C₁₈ alkyl,
in an organic solvent with the following formula:
CH₃-(CH₂)ₘ-CH=CH₂ (II)
where m is an integer value selected from 1 to 20,
and add an amine with the following formula:
NH₂-(CH₂)ₙ₁-CH=CH-(CH₂)ₙ₂-CH₃ (III)
where n1 and n2 are integer values independently selected from between 1 and 10,
b) increasing the temperature of the reaction mixture obtained in (a) to a temperature of between 150-250ºC, subjecting to vacuum and applying radiation with a wavelength of between 430 and 530 nm,
c) breaking the vacuum of the reaction medium of (b) by the introduction of an inert gas,
d) adding to the reaction mixture a reducing agent with the following formula:
R₂-SH (IV)
wherein R₂ is selected from optionally substituted linear or branched C₁-C₁₈ alkyl or optionally substituted C₅-C₁₀ aryl.
e) stopping the reaction and separating the product obtained.

13. Process according to claim 12, wherein R₁ in the bismuth salt of formula (I) is a linear or branched C₈ alkyl.

14. Process according to claim 13, wherein the bismuth salt of formula (I) is as follows:

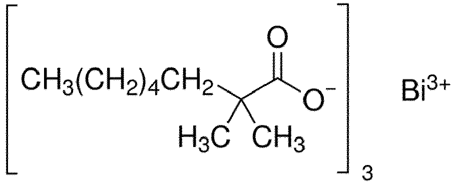

15. Process according to any of claims 12 to 14, wherein m in the organic solvent of formula (II) is an integer value selected from 10 to 18.

16. Process according to the preceding claim, wherein the organic solvent of formula (II) is the following:
CH₃(CH₂)₁₅CH=CH₂

17. Process according to any of claims 12 to 16, wherein n1 and n2 in the amine of formula (III) are an integer value independently selected from between 5 and 8.

18. Process according to the preceding claim, wherein the amine of formula (III) is the following:

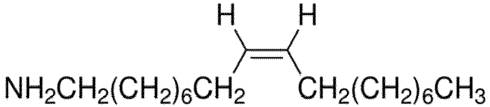

19. Process according to any of claims 12 to 18, wherein the temperature in step (b) is 200°C.

20. Process according to any of claims 12 to 19, wherein the radiation of step (b) is applied for a time of between 10 and 30 minutes, preferably 15 minutes.

21. Process according to any of claims 12 to 20, wherein the inert gas of step (c) is a non-oxidising gas, which is preferably selected from argon, nitrogen or carbon dioxide.

22. Process according to any of claims 12 to 21, wherein R₂ in the reducing agent of formula (IV) is selected from a linear C₈-C₁₂ alkyl or a phenyl.

23. Process according to the preceding claim, wherein the reducing agent of formula (IV) is selected from dodecanethiol or thiophenol.

24. Process according to any one of claims 12 to 23, wherein the reaction is stopped in step (e) by suddenly reducing the temperature of the mixture obtained in (d), preferably by means of an ice bath.

25. Process according to any of claims 12 to 24, wherein the product is separated in step (e) by centrifugation or filtration.

26. Use of the material according to any of claims 1 to 11 for energy storage or generation.

27. Use of the material according to any of claims 1 to 11 for catalysis.

28. Use of the material according to any of claims 1 to 11 for applications in photonics.
